# EUROPEAN PATENT APPLICATION

(11) **EP 2 950 086 A1**
(43) Date of publication of application: **02.12.2015**
(21) Application number: 14170509.5
(22) Date of filing: 29.05.2014
(51) Int. Cl.: G01N 27/02, G01N 33/487

(54) **Device for measuring the trans-layer electrical impedance in an in-vitro model of a cell barrier**

(71) Applicant: Consejo Superior de Investigaciones Cientificas (CSIC), 28006 Madrid (ES); CIBER Centro de Investigación Biomédica en Red, 28029 Madrid (ES)
(72) Inventor: Yeste Lozano, José, 28006 Madrid (ES); Villa Sanz, Rosa, 28006 Madrid (ES); Guimerà Brunet, Anton, 28006 Madrid (ES); Illa Vila, Xavier, 28029 Madrid (ES)
(74) Representative: ZBM Patents - Zea, Barlocci & Markvardsen

(57) **Abstract**

A device mountable in an in vitro model of a cell barrier, the model comprising a first chamber, a second chamber and at least one support for separating the first chamber and the second chamber. The device comprises a first set of electrodes, a second set of electrodes, an element for connecting electrically the device to an electrical apparatus, the element comprising a first joint connected to the first set of electrodes and a second joint connected to the second set of electrodes. The first set of electrodes comprises a first electrode and a second electrode, said electrodes being adapted to be arranged in an interdigitated manner on an inner surface of the first chamber. The second set of electrodes comprises a first electrode and a second electrode, said electrodes being adapted to be arranged in an interdigitated manner on an inner surface of the second chamber.

## Description

The present disclosure relates to a device mountable in an in vitro model of a cell barrier, the model comprising a first chamber, a second chamber and at least one support for separating the first chamber and the second chamber, on which the cells are cultured.

### BACKGROUND ART

In the state of the art it is known that cell barrier (epithelial and endothelial) dysfunction is a hallmark for a great variety of diseases.

For this reason, in recent years there has been an increasing interest in developing models that mimic cell barrier function such as blood-brain barrier. However, these models need to be adequate in order to meet the requirements of high throughput assays and reproducibility. Moreover, real-time monitoring provides an advantageous strategy for achieving a better reproducibility of the models. A common technique for quantitatively evaluate the cell barrier integrity without affecting cell physiology is based on the study of the passive electrical properties of cells. This can be performed by means of electrical impedance spectroscopy (EIS) or transendothelial/epithelial electrical resistance (TEER). Implementation of these measurements in microfluidic systems which better reproduce the in vivo conditions of cell barriers requires new approaches to place the measuring electrodes. Innovative in vitro models, which have become a useful tool to study and mimic the behavior of the barrier to physiological and biochemical changes, as well as the effectiveness of pharmacological treatments, have emerged but few of them allow optical imaging during impedance monitoring due to the area occupied by the electrodes and, thus, compromising the optical inspection of cell cultures, which is still necessary.

This way, for example, with the intention of evaluating the integrity of the same, the trans-layer electrical impedance measure provides a quantificable value of the state of the barrier. "*Trans-layer electrical impedance measure*" means measuring the electrical impedance between the two sides of the barrier.

One of the most common in vitro models to mimic cell barriers consists of two channels, chambers or receptacles separated by at least one support in where cells are cultured. This support may be a porous membrane, where different cell types can be cultured to form the barrier, e.g., one or more types of cells in the same side of the support or, alternatively, in both sides of the support. The inlet and outlet for the medium is placed on the sides of the channels (in dynamic models this medium may have flow).

The most common method to measure the trans-layer electrical impedance in a frequency range of tens of Hertz is to use "Chopstick" external electrodes that are inserted when the measurement is performed. This approach has some disadvantages, among which the most important one is the difficulty to obtain repetitive measures due to the placement of the electrodes not being always the same and the fact of giving a measure where the entire barrier is not equally contributing, but some zones close to the electrodes have a bigger contribution than the remote ones. This phenomenon gets worse when measuring big barrier areas and low trans-layer electrical impedance values.

To solve these disadvantages, there are models with more precise integrated electrodes and which give a more repeatable measure, but which cover a great part of the surface with electrodes and do not allow the optical visualization of the cell barrier.

Thus, for example, the United States patent application US2004152067, entitled "*Impedance based apparatuses and methods for analyzing cells and particles",* describes an apparatus for monitoring cell migration through a biocompatible porous membrane, the apparatus comprising an upper chamber, in which cells whose migration is to be studied are placed; a lower chamber, in which the group of measuring electrodes are located; and a biocompatible membrane with a pore size enough to allow cell migration, which is arranged between both chambers, separating them. The cells under analysis go through the membrane and adhere to the electrodes, modifying the impedance of the group. From the measures of this impedance change, the number of cells that have gone through the cell barrier can be inferred. Therefore, the apparatus is intended to improve the sensitivity in detecting changes in the impedance by means of an elevated quotient between the width of the electrode and the space, which compromises the optical visualization of the cell barrier.

### SUMMARY OF THE INVENTION

The present disclosure provides devices mountable in an in vitro model of a cell barrier, said devices solving at least some of the above-mentioned problems.

In an aspect, a device mountable in an in vitro model of a cell barrier, the model comprising a first chamber, a second chamber and at least one support for separating the first chamber and the second chamber, on which the cells are cultured, comprises:
- a first set of electrodes,
- a second set of electrodes,
- an element for connecting electrically the device to an electrical apparatus, the element comprising at least a first joint connected to the first set of electrodes and at least a second joint connected to the second set of electrodes,
wherein:
- the first set of electrodes comprises a first electrode and a second electrode, said first and second electrodes of the first set of electrodes being adapted to be arranged in an interdigitated manner on an inner surface of the first chamber;
- the second set of electrodes comprises a first electrode and a second electrode, said first and second electrodes of the second set of electrodes being adapted to be arranged in an interdigitated manner on an inner surface of the second chamber.

Consequently, an electrode configuration that minimally covers the culture area is proposed to overcome this limitation. This way, because the interdigitated electrodes only partially cover the cell barrier, it makes the optical visualization of the barrier possible through the space between electrodes.

In some examples, a device for measuring the trans-layer electrical impedance in an in vitro model of a cell barrier, the model comprising a first chamber, a second chamber and at least one support for separating the first chamber and the second chamber, on which the cells are cultured, is provided. The device may comprise a device as described above, wherein the element for connecting electrically the device to an electrical apparatus may comprise an element for connecting electrically the device to an electrical impedance measuring apparatus.

This way, because the interdigitated electrodes only partially cover the cell barrier, it makes the optical visualization of the barrier possible through the space between electrodes using the described configuration of the electrodes (that is, interdigitated electrodes and electrodes in both chambers) allows obtaining a homogeneous sensitivity and reducing the influence of electrode polarization impedance. That is, the use of interdigitated electrodes for performing EIS measurements is proposed as an optimal electrode configuration that allows a similar sensitivity along a defined cell culture area without impairing the optical visualization of the cell culture. Furthermore, the proposed electrode configuration can be adapted to cell culture areas.

In some examples, a device may comprise the electrical impedance measuring apparatus. In this case, the measuring apparatus is a part of the device.

In some examples, a device for applying electroporation in an in vitro model of a cell barrier, the model comprising a first chamber, a second chamber and at least one support for separating the first chamber and the second chamber, on which the cells are cultured, is provided. The device may comprise a device as described above, wherein the element for connecting electrically the device to an electrical apparatus comprises an element for connecting electrically the device to an electroporation apparatus.

This way, it is possible to apply electroporation to the cell barrier. Electroporation is a significant increase in the permeability of the cell plasma membrane caused by exposing the cell to an electric field. In living cells, such permeabilization can be used to enhance the penetration of drugs or DNA plasmids or to destroy undesirable cells.

In some examples, the device may comprise the in vitro model of a cell barrier. In this case, the in vitro model of a cell barrier is a part of the device. Furthermore, in the model, the first chamber may be the upper chamber and the second chamber may be the lower chamber of the in vitro model of a cell barrier.

In said in vitro model of a cell barrier:
- the first chamber of the in vitro model of a cell barrier may comprise a fluid inlet and outlet; and/or
- the second chamber of the in vitro model of a cell barrier may comprise a fluid inlet and outlet.

Said fluid inlets and outlets may be arranged at the end of the chambers or by means of holes in the chambers themselves.

On the other hand, according to some examples, the electrodes may be in contact with an electrical conductor medium, for example an electrical conductor liquid medium, which in many cases may be the culture medium itself. Said electrical conductor medium may be introduced to the chambers through the corresponding fluid inlets.

In some examples, the at least first joint may comprise a first connection point connected to the first electrode of the first set of electrodes, and a second connection point connected to the second electrode of the first set of electrodes. Furthermore, in some examples, the at least second joint may comprise a first connection point connected to the first electrode of the second set of electrodes, and a second connection point connected to the second electrode of the second set of electrodes.

According to this configuration (that is, each joint comprises a first connection point connected to the first electrode and a second connection point connected to the second electrode), it is possible to carry out the measurement of the trans-layer electrical impedance of the cell barrier by means of the technique of impedance measuring to four terminals, in which there are two electrodes where current is injected and another two where the potential is measured. More specifically, for example, the first electrode of the first set of electrodes and the first electrode of the second set of electrodes may be the electrodes where current is injected (current carrying electrodes) while the second electrode of the first set of electrodes and the second electrode of the second set of electrodes may be the electrodes where the potential is measured (pick-up electrodes). By means of this technique of impedance measuring to four terminals the influence of the electrode polarization impedance is reduced.

In this point it is important to highlight that the electrical impedance measuring apparatus may comprise a current source for injecting current to the electrodes and a voltage meter or similar for measuring the potential in the electrodes. Taking into account the value of the injected current and the value of the measured voltage, the apparatus is able to obtain the measure of the trans-layer electrical impedance of the cell barrier.

In another examples, the at least first joint may comprise a single connection point connected to the first and second electrodes of the first set of electrodes. Furthermore, the at least second joint may comprise a single connection point connected to the first and second electrodes of the second set of electrodes.

According to this configuration (that is, each joint only comprises a single connection point connected to the first and second electrodes), it is possible to carry out the measurement of the trans-layer electrical impedance of the cell barrier by means of the technique of impedance measuring to two terminals. Basically, said technique of impedance measuring to two terminals is easier to implement than the technique of impedance measuring to four terminals. The technique of two terminals also requires less instrumentation than the technique of four terminals. However, the technique of two terminals is less accurate than the technique of four terminals because the impedance measures, apart from comprising the trans-layer electrical impedance of the cell barrier, it also comprises the impedance due to the electrode polarization. On the other hand, it is possible to use the technique of two terminals when it is not possible to use the technique of four terminals for example when the impedance of the electrode polarization is many orders of magnitude larger than the impedance of the cell barrier.

According to some examples, the first set of electrodes and the second set of electrodes may be adapted to be arranged facing each other (i.e. there are two pairs of interdigitated electrodes facing each other and located in the surface of the first and second chambers). To visually display the cell barrier, the upper and lower chambers may be transparent. For this reason, inverted microscopes are used, by which the chambers are illuminated from above and the observation is performed from below. This way, the feature relating to the arrangement of the sets of electrodes facing each other allows leaving more space for the observation.

In some examples, each electrode may comprise a plurality of fingers interconnected between them. Said fingers of each electrode may be adapted to be arranged substantially equidistant on the inner surface of the corresponding chamber.

Furthermore, in some examples, the fingers of each electrode may be adapted to be spread throughout the entire surface of the cell barrier.

This way, the entire cell barrier may contribute equally in the impedance measure obtained. Consequently, the device allows the optical visualization through the space between electrodes and allows obtaining an impedance measure where the entire surface of the barrier has a similar contribution.

In some examples, the first electrode and the second electrode of each set of electrodes may comprise the same number of fingers.

According to some examples, each set of electrodes may comprise pairs of fingers, each pair comprising a finger of the first electrode and the neighbouring finger of the second electrode such that each finger of the set of electrodes belongs to a pair and each finger of the set of electrodes belongs to only one pair.

In some examples, the horizontal separation between the fingers of a pair of fingers is substantially equal to the height of the corresponding chamber. The term "substantially equal" may mean that the horizontal separation between the fingers of a pair of fingers may be equal to the height of the corresponding chamber with a variation of ±15%.

According to some examples, the horizontal separation between pairs of fingers is substantially equal to the width of a pair of fingers. The term "substantially equal" may mean that the horizontal separation between pairs of fingers may be equal to the width of a pair of fingers with a variation of ±15%.

This way, taking into account the horizontal separation between the fingers of a pair of fingers and/or the horizontal separation between pairs of fingers, the space free from electrodes could be higher than 50% of the total surface of each chamber.

Additional objects, advantages and features of embodiments of the invention will become apparent to those skilled in the art upon examination of the description, or may be learned by practice of the invention.

### DEFINITIONS

In order to avoid confusions and facilitate understanding of descriptions, this section provides definitions of key concepts in the context of the present disclosure.

The term "*Trans-layer electrical impedance measure"* means measuring the electrical impedance between the two sides of a cell barrier.

The term "*cell barrier*" relates to a layer of cells where adjacent cells are mainly connected by tight junction.

The term *"interdigitated electrodes"* means a plurality of electrodes arranged in interlocking comb-shape, where each electrode comprises an array of parallel fingers.

The term "*electrical conductor liquid medium"* means a liquid able to conduct electrical current through ions.

The term "*joint*" means a place or part at which the electrodes and the electrical apparatus may be joined. Said joint may be physical (e.g. an electrical conductor, a welding or a connector) or logical.

### BRIEF DESCRIPTION OF THE DRAWINGS

Particular examples will be described in the following by way of non-limiting examples, with reference to the appended drawings, in which:
Figure 1 shows a schematic diagram of the device according to the disclosure mounted in an in vitro model of a cell barrier according to some examples;
Figure 2 shows a three-dimensional schematic diagram of the device mounted in an in vitro model of a cell barrier according to some examples;
Figure 3 shows an electrical simulation chart of the sensitivity distribution along a cell barrier for the electrode configuration using the device for measuring the trans-layer electrical impedance in an in vitro model of a cell barrier according to some examples;
Figure 4 shows a simulation chart with the percentage between the total impedance measured and the part corresponding to the trans-layer electrical impedance using the device for measuring the trans-layer electrical impedance in an in vitro model of a cell barrier according to some examples;
Figure 5 shows a schematic diagram of the device for measuring the trans-layer electrical impedance in an in vitro model of a cell barrier according to some examples configured for using the technique of impedance measuring to four terminals;
Figure 6 shows a schematic diagram of the device for measuring the trans-layer electrical impedance in an in vitro model of a cell barrier according to some examples configured for using the technique of impedance measuring to two terminals.

### DETAILED DESCRIPTION OF EMBODIMENTS

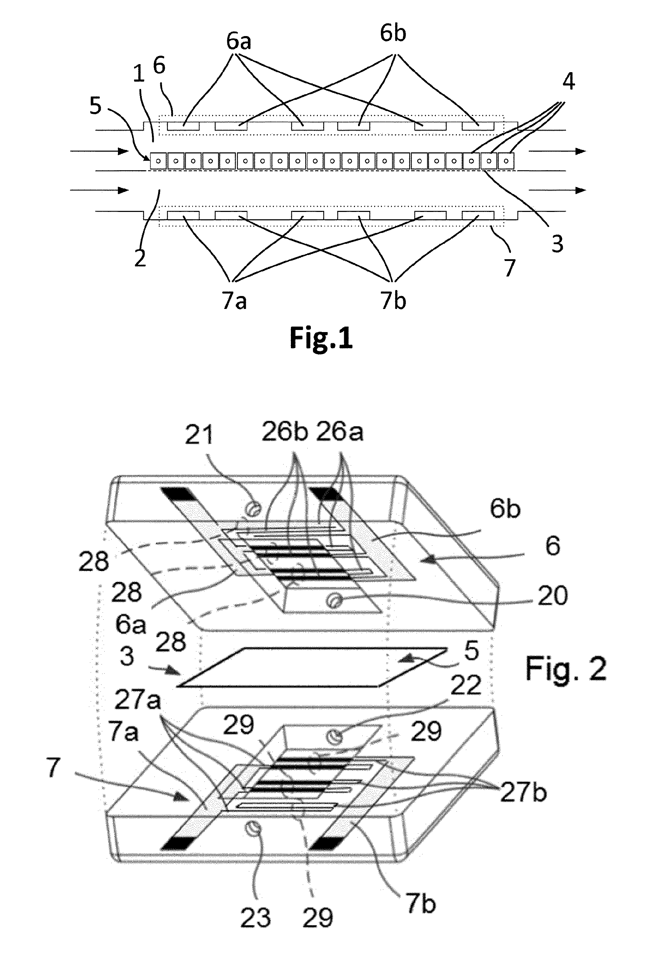

Figure 1 shows an example of a device mounted in an *in vitro* model of a cell barrier. Said device may be used, for example, for measuring the trans-layer electrical impedance in the *in vitro* model of the cell barrier or for applying electroporation to the cell barrier.

As can be seen in Figure 1 the *in vitro* model of the cell barrier may comprise an upper chamber 1 (i.e. a first chamber), a lower chamber 2 (i.e. a second chamber) and a support 3 for separating the upper chamber 1 and the lower chamber 2. On said support 3 cells 4 are cultured for obtaining the cell barrier 5.

According to some examples, the chambers 1,2 may be made of materials with the following features:
- Optically transparent material to visualize the culture of cells;
- Liquid impermeable material;
- Material resistant to culture incubation conditions (typical values 37°C 100% RH);
- biocompatible or non-cytotoxic materials.

On the other hand, the cell barrier 5 may be of different natures. For example, the cell barrier 5 may be:
- A blood_brain barrier. This type of barrier may comprise, for example, three types of cells, that are brain microvascular endothelial cells (BMECs), astrocytes and pericytes;
- Inner blood-retinal barrier. This type of barrier may comprise, for example, retinal capillary endothelial cells.
- Outer blood-retinal barrier. This type of barrier may comprise, for example, retinal retinal pigment epithelial (RPE) cells
- Blood-placental barrier. This type of barrier may comprise, for example, BeWo (clone b30) human placental choriocarcinoma cells.

Furthermore, for introducing an electrical conductor medium (e.g. an electrical conductor liquid medium), each chamber 1,2 (upper and lower) may comprise a fluid inlet and outlet that may be arranged at the end of the chambers or by means of holes in the chambers themselves. According to these examples, the in vitro model comprises a microfluidic system.

It is also possible to see in Figure 1 an example of a device for measuring the trans-layer electrical impedance in the in vitro model of the cell barrier or for applying electroporation to the cell barrier described above, said device being mounted in the in vitro model of the cell barrier. Basically, said device may comprise a first set of electrodes 6 and a second set of electrodes 7 and an element (not shown) for connecting electrically the device, for example, to an electrical impedance measuring apparatus or an electroporation apparatus (not shown), said element having at least a first joint connected to the first set of electrodes 6 and at least a second joint connected to the second set of electrodes 7. This way, said connecting element allows the electrodes to be electrically connected to the electrical impedance measuring apparatus which measure the impedance spectrum or to the electroporation apparatus which allows applying electroporation to the cell barrier.

The first set of electrodes 6 may comprise a first electrode 6a and a second electrode 6b, the first and the second electrodes of the first set of electrodes 6 being adapted to be arranged in an interdigitated manner on the upper inner surface of the upper chamber 1.

The second set of electrodes 7 may comprise a first electrode 7a and a second electrode 7b, the first and the second electrode of the second set of electrodes being adapted to be arranged in an interdigitated manner on the lower inner surface of the lower chamber 2.

Said arrangement of the electrodes in an interdigitated manner partially covers the cell barrier 5 and makes the optical visualization of the barrier possible through the space between electrodes. That is, the use of interdigitated electrodes is proposed as an optimal electrode configuration that allows a similar sensitivity along a defined cell culture area without impairing the optical visualization of the cell culture.

As can be also seen in Figure 1, the electrodes may be in contact with an electrical conductor medium, for example an electrical conductor liquid medium, which in many cases may be the culture medium itself. For this reason, the electrodes may have the following technical features:
- Electrical conductor materials (gold, platinum, silver, carbon, chromium, aluminum, copper, indium tin oxide, titanium, etc.);
- biocompatible or non-cytotoxic materials.

This way, in some examples, the electrodes may be covered with platinum black, PEDOT, polypyrrole, platinum, iridium oxide, carbon nanotubes, graphene, etc.

In some examples the electrodes may be modified to get more roughness, i.e. increase the effective area / geometric area ratio.

On the other hand, Figure 1 shows the first set of electrodes 6 and the second set of electrodes 7 arranged facing each other which allows leaving more space for the observation. To visually display the cell barrier, the upper and lower chambers may be transparent.

Figure 2 shows a three-dimensional schematic diagram of an example of the device mounted in the in vitro model of the cell barrier.

Said figure allows better view of the arrangement of the electrodes of the device. Thus, Figure 2 shows the arrangement of the first and second electrodes 6a,6b of the first set of electrodes 6 in an interdigitated manner on the upper inner surface of the upper chamber 1 and the arrangement of the first and second electrodes 7a,7b of the second set of electrodes 7 in an interdigitated manner on the lower inner surface of the lower chamber 1. Furthermore, Figure 2 shows clearly the first set of electrodes 6 and the second set of electrodes 7 arranged facing each other.

It is also possible to see in Figure 2 the distribution of the electrodes in the in vitro model of the cell barrier, that is, the electrodes may be substantially equidistant and may be spread throughout the entire surface of the cell barrier 5. Basically, each electrode 6a;6b;7a;7b may comprise a plurality of fingers. This way, the first electrode 6a of the first set of electrodes 6 comprises a plurality of fingers 26a, the second electrode 6b of the first set of electrodes 6 comprises a plurality of fingers 26b, the first electrode 7a of the second set of electrodes 7 comprises a plurality of fingers 27a and the second electrode 7b of the second set of electrodes 7 comprises a plurality of fingers 27b.

In this point it is important to highlight that the number of fingers of the first electrode and the second electrode of each set of electrodes may be the same.

The fingers of each electrode may be adapted to be arranged substantially equidistant on the inner surface of the corresponding chamber. Furthermore, in some examples, the fingers of each electrode may be adapted to be spread throughout the entire surface of the cell barrier.

This way, the entire cell barrier may contribute equally in the impedance measure obtained. Consequently, the device allows the optical visualization through the space between electrodes and allows obtaining an impedance measure where the entire surface of the barrier has a similar contribution (i.e. the entire barrier contributes equally in the impedance measure obtained).

Each set of electrodes 6;7 may comprise pairs of fingers 28;29, each pair comprising a finger 26a;27a of the first electrode 6a;7a and the neighbouring finger 26b;27b of the second electrode 6b;7b such that each finger of the set of electrodes belongs to a pair and each finger of the set of electrodes belongs to only one pair.

In some examples, the horizontal separation between the fingers of a pair of fingers is substantially equal to the height of the corresponding chamber. According to some examples, the horizontal separation between pairs of fingers is substantially equal to the width of a pair of fingers. This way, taking into account the horizontal separation between the fingers of a pair of fingers and/or the horizontal separation between pairs of fingers, the space free from electrodes could be higher than 50% of the total surface of each chamber.

Figure 2 also shows a fluid inlet 20 and a fluid outlet 21 in the upper chamber 1 and a fluid inlet 22 and a fluid outlet 23 in the lower chamber 2. In the figure, said fluid inlets and outlets have the form of holes in the chambers themselves.

Consequently, the electrodes may be in contact with an electrical conductor medium, for example an electrical conductor liquid medium, which in many cases may be the culture medium itself. Said electrical conductor medium may be introduced to the chambers through the corresponding fluid inlet.

In summary, an advantage supplied by the proposed device is allowing the optical visualization through the space between electrodes, in addition to obtaining an impedance measure where the entire surface of the barrier has a similar contribution. It also has the advantage of being easily adaptable to different chamber geometries. Additionally, the device allows obtaining a homogeneous sensitivity and reducing the influence of electrode polarization impedance.

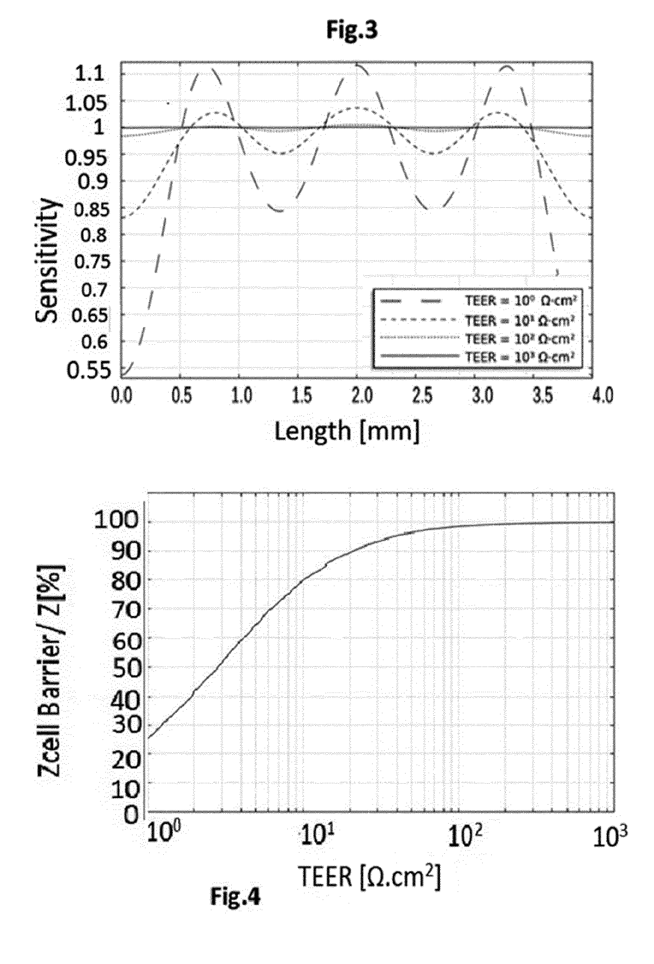

Figure 3 shows an electrical simulation of the sensitivity distribution along the barrier for the electrode configuration of the device for measuring the trans-layer electrical impedance in the in vitro model of the cell barrier by means of the technique of impedance measuring to four terminals. The values are normalized and, therefore, a sensitivity of 1 is the desired value. From the results of the simulation it can be seen that said configuration of the electrodes has a homogenous sensitivity.

Basically, the electrical simulation of Figure 3 is performed taking into account cell barriers with different values of TEER (Trans Epithelial Electric Resistance). A first cell barrier has a TEER value of 10⁰Q·cm² (the cell barrier with the lowest value of TEER, that is, it may be understood as the worse cell barrier used in the simulation), a second cell barrier with a TEER value of 10¹ Ω·cm², a third cell barrier with a TEER value of 10² Ω·cm², and a fourth cell barrier with a TEER value of 10³ Ω·cm² (the cell barrier with the highest value of TEER). As can be seen in Figure 3, in any case, the sensitivity obtained along the cell barrier 5 is homogeneous and near to 1. In order to get a more homogeneous sensitivity, the space between pairs of fingers can be reduced. On the other hand, an increased space between pairs of fingers gets less homogeneous sensitivity but more area of barrier can be visualized.

Figure 4 shows a simulation with the percentage between the total impedance measured and the part corresponding to the trans-layer electrical impedance. The rest of the impedance is due to the medium and to the electrode-electrolyte impedance. A 100% value is the desired value in which the measured impedance is only the trans-layer electrical impedance.

Summarizing, said configuration of the electrodes comprised in the device allows obtaining a homogeneous sensitivity and the medium and electrode-electrolyte interface impedances hardly affect.

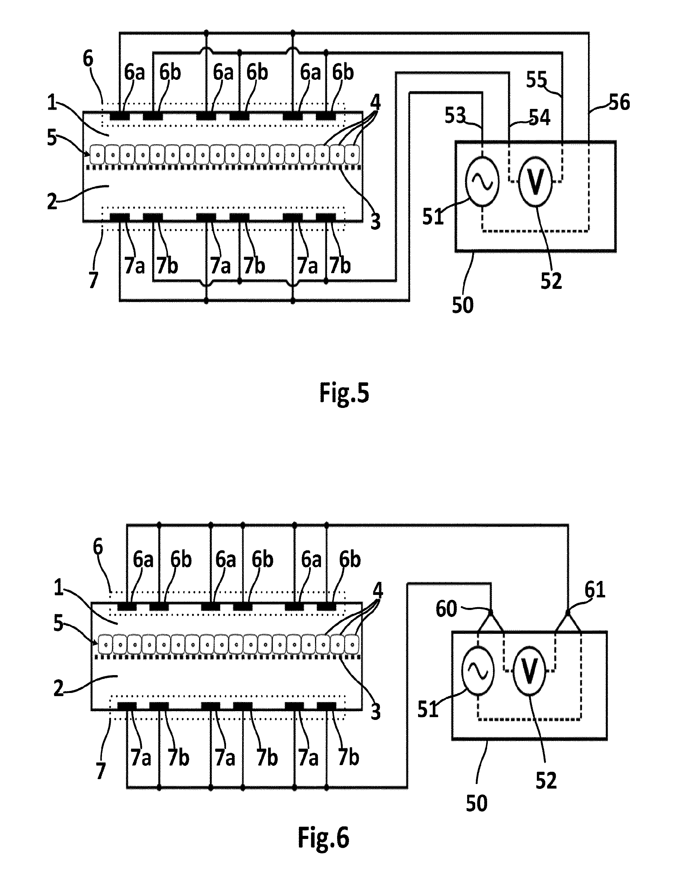

The electrodes may be electrically connected to the electrical impedance measuring apparatus through the connecting element (i.e. the element for connecting electrically the device to the electrical impedance measuring apparatus) described above. Basically, said connection may be performed according to two implementations as can be seen in Figures 5 and 6:
- First implementation (see Figure 5): The at least first joint comprises a first connection point 56 connected to the first electrode 6a of the first set 6 of electrodes, and a second connection point 55 connected to the second electrode 6b of the first set 6 of electrodes. The at least second joint comprises a first connection point 53 connected to the first electrode 7a of the second set 7 of electrodes, and a second connection point 54 connected to the second electrode 7b of the second set 7 of electrodes.
- Second implementation (see Figure 6): The at least first joint comprises a single connection point 61 connected to the first electrode 6a and to the second electrode 6b of the first set 6 of electrodes. The at least second joint comprises a single connection point 60 connected to the first electrode 7a and to the second electrode 7b of the second set 7 of electrodes.

Figure 5 shows the electrical connection of the device to the electrical impedance measuring apparatus 50 according to the first implementation described above. As can be seen in the figure, the electrical impedance measuring apparatus 50 may comprise a current source 51 for injecting current to the electrodes and a voltage meter 52 or similar for measuring the potential in the electrodes. Taking into account the value of the injected current and the value of the measured voltage, the apparatus 50 is able to obtain the measurement of the trans-layer electrical impedance of the cell barrier 5.

Basically, the apparatus 50 may be connected to the device through the connecting element according to the following configuration:
- The first electrode 6a of the first set 6 of electrodes, through the connection point 56, and the first electrode 7a of the second set 7 of electrodes, through the connection point 53, may be connected to the current source 51 of the measuring apparatus 50. This way, there are two electrodes where current is injected. Said electrodes may be referenced as "current injection electrodes" or "current carrying electrodes".
- The second electrode 6b of the first set 6 of electrodes, through the connection point 55, and the second electrode 7b of the second set 7 of electrodes, through the connection point 54, may be connected to the voltage meter 52 of the measuring apparatus 50. This way, there are two electrodes where the potential is measured. Said electrodes may be referenced as "pick-up electrodes" or "potential measuring electrodes".

Consequently, the measurement of the trans-layer electrical impedance may be carried out by means of the technique of impedance measuring to four terminals.

Figure 6 shows the connection of the device to the impedance measuring apparatus 50 according to the second implementation described above.

In this second implementation, the apparatus 50 may be connected to the device through the connecting element according to the following configuration:
- The first electrode 6a and the second electrode 6b of the first set 6 of electrodes may be connected to the current source 51 and to the voltage meter 52 through the connection point 61;
- The first electrode 7a and the second electrode 7b of the second set 7 of electrodes may be connected to the current source 51 and to the voltage meter 52 through the connection point 60.

This way, the potential is measured in the same subsets of electrodes where the current is injected.

Consequently, the measurement of the trans-layer electrical impedance is carried out by means of the technique of impedance measuring to two terminals.

Although only a number of particular embodiments and examples of the invention have been disclosed herein, it will be understood by those skilled in the art that other alternative embodiments and/or uses of the invention and obvious modifications and equivalents thereof are possible. Furthermore, the present invention covers all possible combinations of the particular embodiments described.

Thus, the scope of the present invention should not be limited by particular embodiments, but should be determined only by a fair reading of the claims that follow.

## Claims

1. A device mountable in an in vitro model of a cell barrier, the model comprising a first chamber, a second chamber and at least one support for separating the first chamber and the second chamber, on which the cells are cultured, **characterised in that** the device comprises:
• a first set of electrodes,
• a second set of electrodes,
• an element for connecting electrically the device to an electrical apparatus, the element comprising at least a first joint connected to the first set of electrodes and at least a second joint connected to the second set of electrodes,
wherein:
• the first set of electrodes comprises a first electrode and a second electrode, said first and second electrodes of the first set of electrodes being adapted to be arranged in an interdigitated manner on an inner surface of the first chamber;
• the second set of electrodes comprises a first electrode and a second electrode, said first and second electrodes of the second set of electrodes being adapted to be arranged in an interdigitated manner on an inner surface of the second chamber.

2. A device for measuring the trans-layer electrical impedance in an in vitro model of a cell barrier, the model comprising a first chamber, a second chamber and at least one support for separating the first chamber and the second chamber, on which the cells are cultured, **characterised in that** the device comprises a device according to claim 1, wherein:
• the element for connecting electrically the device to an electrical apparatus comprises an element for connecting electrically the device to an electrical impedance measuring apparatus.

3. A device according to claim 2, further comprising the electrical impedance measuring apparatus.

4. A device for applying electroporation in an in vitro model of a cell barrier, the model comprising a first chamber, a second chamber and at least one support for separating the first chamber and the second chamber, on which the cells are cultured, **characterised in that** the device comprises a device according to claim 1, wherein:
• the element for connecting electrically the device to an electrical apparatus comprises an element for connecting electrically the device to an electroporation apparatus.

5. A device according to any of claims 1 to 4, further comprising the in vitro model of a cell barrier, in which the first chamber is the upper chamber and the second chamber is the lower chamber of the in vitro model of a cell barrier.

6. A device according to claim 5, wherein:
• the first chamber of the in vitro model of a cell barrier comprises a fluid inlet and outlet;
• the second chamber of the in vitro model of a cell barrier comprises a fluid inlet and outlet.

7. A device according to any of claims 5 or 6, wherein the electrodes are in contact with an electrical conductor medium.

8. A device according to any of claims 1 to 7, wherein:
• the at least first joint comprises a first connection point connected to the first electrode of the first set of electrodes, and a second connection point connected to the second electrode of the first set of electrodes;
• the at least second joint comprises a first connection point connected to the first electrode of the second set of electrodes, and a second connection point connected to the second electrode of the second set of electrodes.

9. A device according to any of claims 1 to 7, wherein:
• the at least first joint comprises a single connection point connected to the first and second electrodes of the first set of electrodes;
• the at least second joint comprises a single connection point connected to the first and second electrodes of the second set of electrodes.

10. A device according to any of claims 1 to 9, wherein the first set of electrodes and the second set of electrodes are adapted to be arranged facing each other.

11. A device according to any of claims 1 to 10, wherein the fingers of each electrode are adapted to be arranged substantially equidistant on the inner surface of the corresponding chamber.

12. A device according to any of claims 1 to 11, wherein the fingers of each electrode are adapted to be spread throughout the entire surface of the cell barrier.

13. A device according to any of claims 1 to 12, wherein each set of electrodes comprises pairs of fingers, each pair comprising a finger of the first electrode and the neighbouring finger of the second electrode such that each finger of the set of electrodes belongs to a pair and each finger of the set of electrodes belongs to only one pair.

14. A device according to claim 13, wherein the horizontal separation between the fingers of a pair of fingers is substantially equal to the height of the corresponding chamber.

15. A device according to any of claims 13 or 14, wherein the horizontal separation between pairs of fingers is substantially equal to the width of a pair of fingers.
